(19) Europäisches Patentamt
European Patent Office
Office européen des brevets

(11) Veröffentlichungsnummer: **0 235 826**
**B1**

# (12) EUROPÄISCHE PATENTSCHRIFT

(45) Veröffentlichungstag der Patentschrift: **25.04.90**

(51) Int. Cl.$^5$: **C 07 C 69/708,** A 61 K 6/02

(21) Anmeldenummer: **87103200.9**

(22) Anmeldetag: **06.03.87**

## (54) (Meth)acrylsäureester und ihre Verwendung zur Herstellung von Dentalmassen.

(30) Priorität: **06.03.86 DE 3607331**

(43) Veröffentlichungstag der Anmeldung:
**09.09.87 Patentblatt 87/37**

(45) Bekanntmachung des Hinweises auf die Patenterteilung:
**25.04.90 Patentblatt 90/17**

(84) Benannte Vertragsstaaten:
**AT CH DE FR GB LI**

(56) Entgegenhaltungen:
**DE-A-2 816 823**
**DE-A-2 842 305**
**US-A-4 406 625**

(73) Patentinhaber: **ESPE Stiftung & Co Produktions- und Vertriebs KG**
**Am Griesberg 2**
**D-8031 Seefeld (DE)**

(72) Erfinder: **Schmitt, Werner, Dr.**
**Prinzenweg 10**
**D-8130 Starnberg (DE)**
Erfinder: **Jochum, Peter, Dr.**
**Pointweg 5**
**D-8031 Seefeld 2 (DE)**
Erfinder: **Zahler, Wolf-Dietrich, Dr.**
**An der Beermahd 5**
**D-8031 Seefeld-Hechendorf (DE)**
Erfinder: **Hübner, Heijo, Dr.**
**Moosbichlweg 16**
**D-8031 Wörthsee/Steinebach (DE)**
Erfinder: **Holupirek, Manfred, Dr.**
**Jesenwangerstrasse 2**
**D-8082 Grafrath (DE)**
Erfinder: **Gasser, Oswald, Dr.**
**Hartstrasse 13**
**D-8031 Seefeld (DE)**
Erfinder: **Herzig, Christian, Dr.**
**Mehringerstrasse 80**
**D-8263 Burghausen (DE)**

**EP 0 235 826 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(74) Vertreter: **Abitz, Walter, Dr.-Ing. et al**
**Abitz, Morf, Gritschneder, Freiherr von Wittgenstein Postfach 86 01 09**
**D-8000 München 86 (DE)**

**Beschreibung**

Die Erfindung betrifft neue (Meth)acrylsäureester und ihre Verwendung zur Herstellung von Dentalmassen.

Zur Herstellung von Zahnfüllungen, Zahnversiegelungen sowie von Verblendungen für Kronen, Brücken und Ersatzzähen werden neben Quecksilberamalgam, Gold und Porzellan auch Kunststoffe, insbesondere Polymerisate von leicht polymerisierbaren, olefinisch ungesättigten Verbindungen verwendet. Die Kunststoffe haben gegenüber den anderen Materialien den Vorteil, dass sie weder kosmetisch störend sind noch stoßempfindlich wie Porzellan.

Vor ca. 45 Jahren wurde für diese Zwecke erstmals Poly(methylmethacrylat) verwendet. Bei der Herstellung von Zahnfüllungen kann man die Polymerisation aber nur bei Normal- bzw. Körpertemperatur durchführen. Hierbei bleibt jedoch immer ein kleiner Teil des Methylmethacrylats unpolymerisiert, und es hat sich herausgestellt, dass diese Restmonomeren allmählich aus der Füllung ausdiffundieren und zu Schädigungen, ja meistens zum Pulpentod führen.

In den letzten Jahrzehnten ist man in der Dentalindustrie deswegen auf polyfunktionelle Methacrylsäureester ausgewichen, wie z.B. die in der US—PS 3 066 112 beschriebenen Bis-glycidylmethacrylatester des Isopropylidenphenols, unter Bis-GMA bekannt. Einige Nachteile dieser Monomeren, wie hohe Viskosität und Quellung unter Wasser, konnten durch Modifikation mit Isocyanaten (US—P 3 629 187), durch Verwendung anderer Diphenole oder von Alkadiolen überwunden werden. Biespiele solcher Monomoren sind genannt in DE-PSen 1 921 869, 2 816 823 und 2 934 380 oder den US-PSen 3 629 187 und 4 406 625.

Aus der DE—A 25 41 641 sind strahlenhärtende Bindemittel auf Basis ungesättigter Polyester bekannt, die vorzugsweise in lösemittelfreien Druckfarben verwendet werden sollen.

Alle bisher beschriebenen Monomere erleiden aber bei der Polymerisation einen grossen Polymerisationsschrumpf, so dass ihnen grosse Mengen an anorganischen Füllkörpern beigemengt werdem müssen, um den Schrumpf in Grenzen zu halten. Trotzdem führt der immer noch vorhandene Polymerisationsschrumpf in der Regel zu einem Randspalt zwischen Zahn und Kunststoffüllung oder zwischen Metall und Kunstoffverblendung, so dass zum einen eine schlechte Haftung vorliegt und zum anderen Bakterien, die in den Spalt eindringen, eine Sekundärkaries auslösen können.

Durch die Verwendung der Ätz- und Bonding-Technik oder durch Verwendung von sogenannten Dentinklebern kann der Polymerisationsschrumpf vom Rand der Füllung in die Füllung selbst verlegt werden, so dass trotz eines gewissen Polymerisationsschrumpfes die Haftung zwischen Zahn und Kunstoffüllung oder zwischen Metall und Kunstoffverblendung gewährleistet bleibt. Dieses erfordert aber eine sehr aufwendige Verarbeitungstechnik. Verarbeitungsfehler lassen sich nur mit grösster Sorgfalt verhindern und zeigen sich zum Leidwesen der Patienten erst nach 1 bis 2 Jahren.

Aufgabe der Erfindung ist die Bereitstellung neuer (Meth)acrylsäureesster, die zur Herstellung von schrumpfarmen Dentalmassen verwendet werden können.

Diese Aufgabe wird erfindungsgemäss durch die Schaffung von (Methy)acrylsäureestern der allgemeinen Formel

$$(MO)_n\!\left[A-O-CO-CH_2-O-CH_2-CH_2-O-CH_2-CO-O\right]_x\!A-(OM)_n \qquad I$$

gelöst, worin bedeuten

A einen Alkoholrest mit 4 bis 30 Kettenatomen zwischen zwei Verknüpfungsstellen, wobei die Kettenatome C- oder O-Atome sind,

M $CH_2=C(R^1)-CO-$,

wobei $R^1$ Wasserstoff oder Methyl bedeutet,

n 1 oder 2,

x 0,3 bis 3.

Vorzugsweise bedeutet x 0,5 bis 2.

Die erfindungsgemässen (Meth)acrylsäureester liegen vorzugsweise als Gemische von Verbindungen der allgemeinen Formel vor, in der x verschiedene Werte zwischen 1 und 3 hat, insbesondere ein Gemisch von zwei Verbindungen mit x = 1 und 2 im Molverhältnis 1:1.

Der Rest A hat vorzugsweise mindestens 8 Kettenatome zwischen zwei Verknüpfungsstellen.

Die Ketten zwischen zwei Verknüpfungsstellen können Seitengruppen aufweisen.

Geeignete Alkohole $A(OH)_{n+1}$ sind beispielsweise Alkandiole, Cycloalkandiole, alkoxylierte Cycloalkandiole sowie Bisphenol-A-Derivate, insbesondere alkoxylierte Bispheol-A-Derivate. Bevorzugt sind n-Dodecyl-1,12-diol, die Bis-(2-hydroxyethylether) und Bis-(2-hydroxypropylether) des 2,2-Isopropylidendiphenols, insbesondere Bis-(hydroxymethyl)-tricyclo-[5.2.1.$0^{2,6}$]-decan.

Der Alkohol $A(OH)_{n+1}$ ist verestert, einerseits mit Triglykolsäure, andererseits mit Acrylsäure oder Methacrlysäure.

Beispiele erfindungsgemässer Monomeren sind Triglykolsäure-bis[3(4)-methacryloxymethyl-8(9)-tricyclo-[5.2.1.$0^{2,6}$]-decylmethylester], Triglykolsäure-bis-[3(4)-acryloxymethyl-8(9)-tricyclo[5.2.1.$0^{2,6}$]-decylmethylester], Triglykolsäure-bis[octyloxymethacrylat], Triglykolsäure-bis]dodecyloxymethacrylat] und Triglykolsäure-bis[4-methacryloxyethoxy-4-2,2-diphenylpropylester].

Die erfindungsgemässen Verbindungen und Verbindungsgemische sind im allgemeinen mässig viskose Flüssigkeiten oder relativ niedrig schmelzende Substanzen. Sie werden in an sich bekannter Weise durch die üblichen Methoden der Veresterung oder Umesterung hergestellt. So können beispielsweise die Polyole direkt mit Triglykolsäure un Gegenwart der bekannten Veresterungskatalysatoren, wie Paratoluolsulfonsäure, teilverestert werden. Die restlichen Hydroxylfunktionen des Polyols werden unter Zugabe von üblichen Polymerisationsinhibitoren, beispielsweise Pikrinsäure, Methylenblau und Ionol, mit Methacrylsäure oder Acrylsäure verestert.

Die erfindungsgemässen Ester können in für (Meth)acrylsäureester üblicher Weise durch Radikalbildner leicht polymerisiert werden.

Geeignete Initiatorsysteme sind z.B. die bekannten Redox-Systeme. Mit Hilfe dieser Initiatoren können die erfindungsgemässen Ester in wenigen Minuten bei Raum- oder Körpertemperatur auspolymerisiert werden. Vorzuziehen sind Systeme, welche nicht zur Verfärbung neigen, z.B. die bekannten Sulfonderivate, wie Hydroxy-oder Aminosulfone. Auch lösliche Salze von Sulfinsäuren mit tertiären Aminen oder quartären Ammoniumbasen, insbesondere in Kombination mit Peroxiden, sind geeignet. Auch sogenannte CH-aktive Substanzen, wie in 5-Stellung monosubstituierte Barbitursäure-derivate oder β-Diketonderivate, können, in Kombination mit den bekannten Cokatalysatoren, wie Schwermetallspuren und Chloridionen, verwendet werden.

Weitere Radikalbildner sind z.B. aus der deutschen Patentschrift 942 540, der französischen Patentschrift 981 085 und den deutschen Auslegeschriften 1 037 132, 065 617 und 1 217 063 bekannt. Als Peroxide sind beispielsweise geeignet: Benzoylperoxid, Lauroylperoxid, Mono-t-Butylpermaleinat oder t-Butyl-hydroperoxid. Bei Azocarbonsäurenitrilen kommt unter anderem Azo-Isobutyronitril infrage.

Ein anderes, besonders geeignetes Initiatorsystem ist ein Photoinitiatorsystem; Photoinitiatoren sind beispielsweise in den deutschen Offenlegungsschriften 2 251 048 und 2 003 132 sowie in den US—PSen 2 548 685, 2 495 987, 3 551 246 und 3 558 387 sowie in der europäischen Patentveröffentichungsschrift 0 073 413 beschrieben. Als Beispiele seien erwähnt Benzoinether, Benzophenone und Diketone, z.B. Campherchinon oder Benzil. Die Photoinitiatoren können gegebenefalls zusammen mit Aktivatoren verwendet werden. Geeignete Aktivatoren sind z.B. Amine, insbesondere tertiäre Amine, und die beispielsweise in der deutschen Offenlegungsschrift 2 251 048 genannten Aktivatoren (Reduktionsmittel). Der Photoinitiator wird im allgemeinen in einer Menge von 0,01 bis 10 Gew.-%, bezogen auf die polymerisierbare Monomermischung, formuliert.

Die erfindungsgemässen Verbindungen oder Verbindungsgemische können allein oder zusammen mit anderen polymerisierbaren, ethylenisch ungesättigten Monomeren zur Herstellung von Dentalmassen eingesetzt werden. Dies kann im Einzelfall zur Erreichung der gewünschten Viskosität des Monomerengemischs sinnvoll sein. Besonders bei erfindungsgemässen Monomeren der allgemeinen Formel mit Kettenlängen $z > 1$ und Alkoholresten A mit mehr als 8 Kettenatomen ist es sinnvoll, diese zusammen mit niedrig viskosen Monomeren einzusetzen.

Geeignete niedrig viskose Monomere sind beispielsweise die Acrylsäure- und Methacrylsäureester mono- oder polyfunktioneller Alkohole. Solche Alkohole sind beispielsweise solche der allgemeinen Formel $A(OH)_{n+1}$. Zusätzlich sind auch Acrylsäure- und Methacrylsäureester mono- oder polyfunktioneller Alkohole mit Kettengliedern $< 4$ geeignet, etwa Methanol, Ethanol und Ethylenglykol. Vorzugsweise ist der Alkoholteil der niedrig viskosen Monomeren gleich dem in den erfindungsgemässen Verbindungen verwendeten. Ganz besonders bevorzugte niedrig viskose Monomere sind die Acrylsäure- und Methacrylsäureester des Bis-(hydroxymethyl)-tricyclo[5.2.1.$0^{2,6}$]-decans.

Zur Polymerisation und Härtung auf der Zahnoberfläche oder in einer Zahnkavität muss die aus den erfindungsgemässen (Meth)acrylsäureestern hergestellte Dentalmasse ausserdem einen oben erwähnten Initiator, z.B. ein Peroxid, wie Benzoylperoxid, und gegebenenfalls einen Aktivator, z.B. ein tertiäres Amin, wie N,N-Dimethyl-p-Toluidin, enthalten. Ein derartiges Härtungssystem wird in einer Menge von 0,1 bis 6 Gew.-%, bezogen auf die polymerisierbare Monomermischung, zugegeben.

Hierzu besteht die polymerisierbare Dentalmasse aus wenigstens zwei getrennten Packungen, von denen die eine den Initiator und die andere den Aktivator enthält. Vor der Verwendung werden die beiden Packungsinhalte vermischt.

Bei der Herstellung von Zahnersatzteilen mit Lauroylperoxid erhitzt man zweckmässigerweise die Masse in der Form einige Stunden auf 90°C oder kurze Zeit auf Temperaturen bis etwa 160°C, vorzugsweise auf 120°C bis 160°C.

Zur Herstellung von Dentalmassen setzt man den erfindungsgemässen Monomeren ausserdem zweckmässigerweise Füllstoffe in einer Konzentration von 20—95%, bevorzugt 40—85% zu. Als Füllstoffe geeignet sind beispielsweise feinzerteiltes Polymethylmethacrylat, Glas- oder Quarzfasern Quarzmehl, Aluminiumoxid, Silikate, pyrogene Kieselsäure, Kieselgele, oder aus den letzten beiden Produkten hergestellte Agglomerate oder Granulate. Vorteilhaft ist ferner der Zusatz von Pigmenten, um eine zahnähnliche Einfärbung des polymerisierbaren Dentalmaterials zu erreichen.

Es kann weiterhin vorteilhaft sein, der Dentalmasse röntgenopake Zusatzstoffe zuzusetzen, beispielsweise Bariumsulfat, Zirkondioxid, oder Barium- und Strontium-gläser. Diese Stoffe können dem Füllstoff in einer Konzentration von 1—100%, bezogen auf den Füllstoffgehalt, zugegeben werden.

Besonders vorteilhaft ist die Verwendung von mit Haftvermittlern vorbehandelten Füllstoffen. Geeignete Haftvermittler sind z.B. Silane, wie Trimethoxy-methacrylpropoxysilan.

Die unter Verwendung der erfindungsgemässen Ester erhaltenen Dentalmassen zeigen überraschenderweise einen deutlich geringeren Polymerisationsschrumpf als die mit bekannten bi- oder polyfunktionellen Monomeren hergestellten Dentalmassen. Besonders vorteilhaft ist die Verwendung der erfindungsgemässen Ester in Dentalmassen, in die nur wenige anorganische Füllkörper eingebracht werden können, z.B. in mit sogenannten Mikrofüllern hergestellten Dentalmassen. Diese enthalten in der Regel weniger als 70 Gew.-% anorganische Füllkörper. Im Gegensatz zu den sogenannten "Makrofüller-Dentalmassen" lassen sich die genannten "Makrofüller-Dentalmassen" polieren, so dass mit ihnen "kosmetisch perfekte" Zahnfüllungen und -verblendungen hergestellt werden können. Die mit bekannten Monomeren hieraus hergestellten Dentalmassen zeigen aber nach der Erhärtung einen deutlichen Polymerisationsschrumpf.

Mit den aus erfindungsgemässen Monomeren hergestellten Dentalmassen erhält man also mit weniger Füllstoff die gleichen guten Schrumpfeigenschaften, wie sie herkömmlicherweise nur mit sehr hochgefüllten Präparaten zu erreichen waren.

Beispiel 1 (Herstellungsbeispiel)

Triglykolsäure-bis[3(4)-methacryloxymethyl-8(9)-tricyclo[$5.2.1.0^{2,6}$]decylmethylester].

196 g (1,0 Mol) Bis-(hydroxymethyl)-tricyclo[$5.2.1.0^{2,6}$]-decan (T-diol), gelöst in 400 ml Cyclohexan, werden unter Ausschleppen von 18 g Wasser mit 89 g (0,5 Mol) Triglykolsäure teilverestert. Als Katalysator verwendet man 7 g p-Toluolsulfonsäure. Die restlichen Hydroxylfunktionen des Diols werden unter Zugabe von 80 mg Pikrinsäure, 200 mg Methylenblau und 35 mg Ionol als Polymerisationsinhibitoren mit 129 g (1,5 Mol) frisch destillierter Methacrylsäure verestert, wobei sich weitere 18 g Wasser abscheiden. Das rohe Estergemisch verdünnt man mit weiteren 400 ml Cyclohexan und 250 ml Toluol und wäscht mit 4 × 350 ml 2-n NaOH, 2 × 350 ml 0,5-n $H_2SO_4$ sowie mit Wasser. Man trocknet die Esterlösung mit Natriumsulfat, durchperlt sie mit Luftsauerstoff und reinigt abschliessend über Aluminiumoxid. Nach Zugabe von 4-Methoxyphenol zur Inhibierung erhält man durch Konzentrieren im Hochvakuum 258 g eines Estergemisches folgender durchschnittlicher Zusammensetzung:

32% T-diol-bis-methacrylat
38% Triglykolsäure-bis[T-methacrylat]
30% Bis-(methacrylkat) von

$$OH-[T-O-CO-CH_2-O-CH_2-CH_2-O-CH_2-CO-O]_{3-3}-T-OH$$

Viskosität: 80 poise
Dichte: 1,152 g/ml
$n_p^{20}$: 1,496

'H—NMR: 0,9—2,7 ppm (Tricyclus)
1,97 ppm ($CH_3$—C=C)
3,77 ppm (O—$CH_2$—$CH_2$—O)
3,7—4,2 ppm (tricyclo-$CH_2$—O)
4,17 ppm (O—$CH_2$—$CO_2$—)
5,54 ppm (trans-CH=C—C=O)
6,09 ppm (CiS—CH=C—O)

IR (Film): τ (C—H) = 2940, 2870 $cm^{-1}$
τ (C=O) = 1751, 1715 $cm^{-1}$
τ (C=C) = 1635 $cm^{-1}$
τ (C—O) = 1196, 1160, 1130 $cm^{-1}$

Beispeil 2 (Verwendungsbeispiel)

50 Gewichtsteile Bis-(acryloxymethyl)-tricyclo[$5.2.1.0^{2,6}$]-decan und 50 Gewichtsteile des im Beispiel 1 erhaltenen Estergemisches werden unter vorsichtigem Erwärmen so lange gerührt, bis eine klare Lösung entsteht.

Zu der auf Raumtemperatur abgekühlten Lösung gibt man 0,15 Gew-.% Campherchinon und 1,5 Gew.-% N,N-Dimethylaminoethylmethacrylat und rührt so lange, bis eine klare Losung vorliegt.

112 Gewichtsteile silanisiertes Kieselsäuregranulat (EP—PS 0 040 232), 72 Gewichsteile silanisierte, pyrogene Kieselsäure (Aerosil OX 50 von Degussa) und 3 g Calciumfluorid werden zu einem homogenen Pulver vermischt und zahnähnlich eingefärbt.

113 g dieses Pulvers werden in kleinen Portionen zu 80 g der Lösung hinzugegeben und zu einer Zahnfüllmasse mit einheitlicher, pastöser Konsistenz verknetet.

Füllt man die erhaltene Zahnfüllmasse in einen Zylinder (Durchmesser 5 mm, Länge 8 mm), belichtet 20 Sekunden mit einem handelsüblichen dentalen Bestrahlungsgerät (Elipar-Visio/ESPE), nimmt anschliessend das Polymerisat aus dem Zylinder und entfernt die weichen oder gelartigen, nicht durchpolymerisierten Bestandteile mit einem Kunststoffspatel, so erhält man eine durchpolymerisierte Schicht von 3,4 mm Dicke. Die Druckfestigkeit beträgt 431,5 $N/mm^2$.

Beispiel 3 (Verwendungsbeispiel)

In extrahierte Rinderschneidezähne werden labial zylinderische Kavitäten mit einem Durchmesser von 3 mm in den Zahnschmelz präpariert. Die präparierten Kavitäten hatten eine Tiefe von 2,5 mm.

In diese Kavitäten werden die zu prüfenden Zahnfüllmassen eingebracht und 20 Sekunden mit einem handelsüblichen dentalen Bestrahlungsgerät (Elipar-Visio/ESPE) ausgehärtet.

Als Zahnfüllmassen werden die in Beispiel 2 beschriebene Masse und eine Masse verwendet, die die gleiche Füllkörperzusammensetzung, aber eine für Zahnfüllmassen gebräuchliche Monomerischung aus 45 Teilen Bis-(acryloxymethyl)-tricyclo[5.2.1.0$^{2,6}$]-decan, 45 Teilen Bis-(methacryloxymethyl)-tricyclo[5.2.1.0$^{2,6}$]-decan und 10 Teilen Bis-GMA enthält (Visio-Dispers, Espe).

Anhand eines zur Füllungsoberfläche senkrechten Schnittes durch den Rinderzahn wird die Randspaltbildung am Boden, in der Mitte und am oberen Rand an jeweils 5 Messzähnen mit Hilfe eines Mikroskopes vermessen. In der nachstehenden Tabelle sind die Mittelwerte der Messungen aufgelistet.

TABELLE

| | Polymerisationsschrumpf (µm) | | |
|---|---|---|---|
| Zahnfüllmasse | in der Mitte | am Kavitätenboden | an oberen Rand |
| nach Beispiel 2 | 3 ± 2,3 | 7,0 ± 2,3 | 2 ± 1,1 |
| Vergleichspräparat (Visio-Dispers, Espe) | 12,3 + 5,3 | 21,0 ± 9,1 | 4,8 ± 5,6 |

Deutlich ist der um den Faktor 2—3 geringere Polymerisationsschrumpf der Zahnfüllmasse, die die erfindungsgemässe Monomermischung enthält, gegenüber einem mit gleichen Füllkörpern gefüllten bekannten Vergleichspräparat zu sehen.

**Patentansprüche für die Vertragsstaaten: BE CH DE FR GB IT LI LU NL SE**

1. (Meth)acrylsäureester der allgemeinen Formel

$$(MO)_n \overline{+} A—O—CO—CH_2—O—CH_2—CH_2—O—CH_2—CO—O \overline{+}_x A—(OM)_n \quad I$$

worin bedeuten

A einen Alkoholrest mit 4 bis 30 Kettenatomen zwischen zwei Verknüpfungsstellen, wobei die Kettenatome C- oder O-Atome sind,

M $CH_2{=}C(R^1)—CO—$,

wobei $R^1$ Wasserstoff oder Methyl bedeutet,

n 1 oder 2,

x 0,3 bis 3.

2. Verbindungen nach Anspruch 1, dadurch gekennzeichnet, dass n 1 bedeutet.

3. Verbindungen nach Anspruch 1 oder 2, dadurch gekennzeichnet, dass x 0,5 bis 2 bedeutet.

4. Verbindungen nach einem der Ansprüche 1 bis 3, dadurch gekennzeichnet, dass A mindestens 8 Kettenatome aufweist.

5. Verbindungen nach einem der Ansprüche 1 bis 4, dadurch gekennzeichnet, dass sie als Alkoholreste Reste (a) des n-Dodecyl-1,12-diols, (b) des Bis-(2-hydroxyethylethers) oder Bis-(2-hydroxypropylethers) des 2,2-Isopropylidendiphenols oder (c) des Bis-(hydroxymethyl)-tricyclo[5.2.1.0$^{2,6}$]-decans enthalten.

6. Verwendung von Verbindungen nach einem der Ansprüche 1 bis 5 zur Herstellung von Dentalmassen.

7. Verwendung eines Gemisches von zwei Verbindungen nach einem der Ansprüche 2 bis 5 im Molverhältnis 1:1, die sich dadurch unterscheiden, dass bei der einen Verbindung x = 1 und bei der anderen Verbindung x = 2 ist, zur Herstellung von Dentalmassen.

8. Verwendung von Verbindungen nach Ansprüche 6 oder 7 in Kombination mit polymerisierbaren, ethylenisch ungesättigten Monomeren, insbesondere mit Estern aus (Meth)acrylsäure und mono- oder polyfunktionellen Alkoholen, insbesondere der allgemeinen Formel $A(OH)_{n+1}$.

**Patentansprüche für den Vertragsstaat: AT**

1. Verwendung von (Meth)acrylsäureestern zur Herstellung von Dentalmassen, dadurch gekennzeichnet, dass die (Meth)acrylsäureester die allgemeine Formel

$$(MO)_n \overline{+} A—O—CO—CH_2—O—CH_2—CH_2—O—CH_2—CO—O \overline{+}_x A—(OM)_n \quad I$$

aufweisen, worin bedeuten

A einen Alkoholrest mit 4 bis 30 Kettenatomen zwischen zwei Verknüpfungsstellen, wobei die Kettenatome C- oder O-Atome sind,

M $CH_2{=}C(R^1){-}CO{-}$,

wobei $R^1$ Wasserstoff oder Methyl bedeutet,

n 1 oder 2,

x 0,3 bis 3.

2. Verwendung nach Anspruch 1, dadurch gekennzeichnet, das n in der allgemeinen Formel I den Wert 1 hat,

3. Verwendung nach Anspruch 1 oder 2, dadurch gekennzeichnet, das x in der allgemeinen Formel I den Wert 0,5 bis 2 hat.

4. Verwendung nach einem der Ansprüche 1 bis 3, dadurch gekennzeichnet, dass A in der allgemeinen Formel I mindestens 8 Kettenatome aufweist.

5. Verwendung nach einem der Ansprüche 1 bis 4, dadurch gekennzeichnet, dass die verwendete Verbindung als Alkoholrest den Rest (a) des n-Dodecyl-1,12-diols, (b) des Bis-(2-hydroxyethylethers) oder des Bis-(2-hydroxypropylethers) des 2,2-Isopropylidendiphenols oder (c) des Bis-(hydroxymethyl)-tricyclo[5.2.1.$0^{2,6}$]-decans enthält.

6. Verwendung nach einem der Ansprüche 2 bis 5, dadurch gekennzeichnet, dass ein Gemish von zwei Verbindungen im Molverhältnis 1:1 verwendet wird, die sich dadurch unterscheiden, dass bei der einen Verbindung x = 1 und bei der anderen Verbindungen x = 2 ist.

7. Verwendung nach einem der Ansprüche 1 bis 6, dadurh gekennzeichnet, dass sie in Kombination mit polymerisierbaren, ethylenisch ungesätigten Monomeren, insbesondere mit Estern aus (Meth)acrylsäure und mono- oder polyfunktionellen Alkoholen, insbesondere der allgemeinen Formel $A(OH)_{n+1}$ erfolgt.

8. Verfahren zur Herstellung von (Meth)acrylsäureestern der allgemeinen Formel

$$(MO)_n{+}A{-}O{-}CO{-}CH_2{-}O{-}CH_2{-}CH_2{-}O{-}CH_2{-}CO{-}O{+}_x A{-}(OM)_n \qquad I$$

worin bedeuten

A einen Alkoholrest mit 4 bis 30 Kettenatomen zwischen zwei Verknüpfungsstellen, wobei die Kettenatome C- oder O-Atome sind,

M $CH_2{=}C(R^1){-}CO{-}$,

wobei $R^1$ Wasserstoff oder Methyl bedeutet,

n 1 oder 2,

x 0,3 bis 3,

dadurch gekennzeichnet, dass man ein Polyol der allgemeinen Formel

$$A(OH)_{n+1}$$

worin n die Zahl 1 oder bedeutet und A einen Alkoholrest mit 4 bis 30 Kettenatomen zwischen zwei Verknüpfungsstellen darstellt, und die Kettenatome C-oder O-Atome sind, mit Triglykolsäure der Formel

$$HOOC{-}CH_2{-}O{-}CH_2{-}CH_2{-}O{-}CH_2{-}COOH$$

in Gegenwart eines Veresterungskatalysators teilverestert und die restlichen Hydroxylgruppen des Polyols in Gegenwart eines Polymerisationsinhibitors mit Acrylsäure oder Methacrylsäure verestert.

9. Verfahren nach Anspruch 8, dadurch gekennzeichnet, das in der allgemeinen Formel I n den Wert 1 und x den Wert 0,5 bis 2 haben.

10. Verfahren nach Anspruch 8 oder 9, dadurch gekennzeichnet, dass A in der allgemeinen Formel I mindestens 8 Kettenatome aufweist.

**Revendications pour les Etats contractants: CH DE FR GB LI**

1. Esters de l'acide (méth)acrylique, de formule générale

$$(MO)_n{+}A{-}O{-}CO{-}CH_2{-}O{-}CH_2{-}CH_2{-}O{-}CH_2{-}CO{-}O{+}_x A{-}(OM)_n \qquad I$$

dans laquelle

A est un résidu alcool ayant dans sa châne de 4 à 30 atomes entre deux points de jonction, les atomes de chaîne étant des atomes de carbone ou d'oxygène,

M est le radical $CH_2{=}C(R^1){-}CO{-}$, $R^1$ étant un hydrogène on le radical méthyle,

n vaut 1 ou 2,

x vaut de 0,3 à 3.

2. Composés selon la revendication 1, dans laquelle n vaut 1.

3. Composés selon la revendication 1 ou 2, caractérisés en ce que x vaut de 0,5 à 2.

4. Composés selon l'une des revendications 1 à 3, caractérisé en ce que A comporte au moins 8 atomes de chaîne.

5. Composés selon l'une des revendications 1 à 4, caractérisés en ce qu'ils contiennent en tant que radicaux alcool des résidus (a) du n-dodécyl-1,12-diol, (b) de l'ether bis-(2-hydroxyéthylique) ou bis-(2-hydroxypropylique) du 2,2-isopropylidènediphénol, ou (c) du bishydroxyméthyl)-tri-cyclo[5.2.1.$0^{2,6}$]-décane.

6. Utilisation de composés selon l'une des revendications 1 à 5 pour la préparation de masses dentaires.

7. Utilisation d'un mélange de deux composés selon l'une des revendications 2 à 5 selon un rapport en moles de 1:1, qui se distinguent l'un de l'autre par le fait que x = 1 pour l'une des composés et x = 2 pour l'autre, pour la préparation de masses dentaires.

8. Utilisation de composés selon les revendications 6 à 7 en combinaison avec des monomères polymérisables à insaturation éthylénique, en particulier des esters de l'acide (méth)acrylique et de mono- ou de polyalcools, en particulier de formule générale $A(OH)_{n+1}$.

**Revendications pour l'Etat contractant: AT**

1. Utilisation d'esters de l'acide (méth)acrylique pour la préparation de masses dentaires, caractérisée en ce que les esters de l'acide (méth)acrylique ont la formule générale

$$(MO)_n\text{—}[A\text{—}O\text{—}CO\text{—}CH_2\text{—}O\text{—}CH_2\text{—}CH_2\text{—}O\text{—}CH_2\text{—}CO\text{—}O]_x\text{—}A\text{—}(OM)_n \qquad I$$

dans laquelle

A est un résidu alcool ayant dans sa chaîne de 4 à 30 atomes entre deux points de jonction, les atomes de chaîne étant des atomes de carbone ou d'oxygène,

M est le radical $CH_2{=}C(R^1)\text{—}CO\text{—}$, $R^1$ étant un hydrogène ou le radical méthyle,

n vaut 1 ou 2,

x vaut de 0,3 à 3.

2. Composés selon la revendication 1, dans laquelle n, dans la formule générale I, vaut 1.

3. Composés selon la revendication 1 ou 2, caractérisés en ce que x, dans la formule générale I, vaut de 0,5 à 2.

4. Composés selon l'une des revendications 1 à 3, caractérisés en ce que A, dans la formule générale I, comporte au moins 8 atomes de chaîne.

5. Utilisation selon l'une revendications 1 à 4, caractérisé en ce que le composé utilisé contient en tant que radical alcool le résidu (a) du n-dodécyl-1,12-diols, (b) de l'ether bis-(2-hydroxyéthylique) ou bis-(2-hydroxypropylique) du 2,2-isopropylidènediphénol, ou (c) du bis-(hydroxyméthyl)-tricyclo[5.2.1.$0^{2,6}$]-décane.

6. Utilisation selon l'une des revendications 2 à 5, caractérisée en ce qu'on utilise un mélange de deux composés, selon un rapport en moles de 1:1, qui se distinguent l'un de l'autre par le fait que x = 1 dans un composé et x = 2 dans l'autre.

7. Utilisation selon l'une des revendications 1 à 6, caractérisée en ce qu'elle est mise en oeuvre en combinaison avec des monomères polymérisables à insaturation éthylénique, en particulier avec des esters de l'acide (méth)acrylique et des mono- ou polyalcools, en particulier de formule générale $A(OH)_{n+1}$.

8. Procédé de préparation d'esters de l'acide (méth)acrylique, de formule générale

$$(MO)_n\text{—}[A\text{—}O\text{—}CO\text{—}CH_2\text{—}O\text{—}CH_2\text{—}CH_2\text{—}O\text{—}CH_2\text{—}CO\text{—}O]_x\text{—}A\text{—}(OM)_n \qquad I$$

dans laquelle

A est un résidu alcool ayant dans sa chaîne de 4 à 30 atomes entre deux points de jonction, les atomes de chaîne étant des atomes de carbone ou d'oxygène,

M est le radical $CH_2{=}C(R^1)\text{—}CO\text{—}$, $R^1$ étant un hydrogène ou le radical méthyle,

n vaut 1 ou 2,

x vaut de 0,3 à 3,

caractérisé en ce qu'on fait subir une estérification partielle, en présence d'un catalyseur d'estérification, à un polyol de formule générale

$$A(OH)_{n+1}$$

dans laquelle n vaut 1 ou 2, et A est un radical alcool ayant de 4 à 30 atomes de chaîne entre deux points de jonction, les atomes de chaîne étant des atoms de carbone ou d'oxygène, avec de l'acide triglycolique de formule

$$HOOC\text{—}CH_2\text{—}O\text{—}CH_2\text{—}CH_2\text{—}O\text{—}CH_2\text{—}COOH$$

et qu'on estérifie les groupes hydroxyle restants du polyol en présence d'un inhibiteur de polymérisation, avec de l'acide acrylique ou méthacrylique.

9. Procédé selon la revendication 8, caractérisé en ce que, dans la formule générale I, n vaut 1 et x vaut de 0,5 à 2.

10. Procédé selon la revendication 8 ou 9, caractérisé en ce que A, dans la formule générale I, comporte au moins 8 atomes de chaîne.

**Claims for the Contracting States: CH DE FR GB LI**

1. (Methy)acrylic acid esters of the general formula

$$(MO)_n\!-\!\!\left[A—O—CO—CH_2—O—CH_2—CH_2—O—CH_2—CO—O\right]_x\!A—(OM)_n \qquad \text{I}$$

wherein

A represents an alcohol radical with 4 to 30 chain-atoms between two points of linkage, where the chain-atoms are carbon or oxygen atoms,

M $CH_2{=}C(R^1)$-CO-, where $R^1$ represents hydrogen or methyl,

n represents 1 or 2,

x represents 0.3 to 3.

2. Compositions according to claim 1, characterized in that n represents 1.

3. Compositions according to claim 1 or 2, characterized in that x represents 0.5 to 2.

4. Compositions according to one of claims 1 to 3, characterized in that A has at least 8 chain-atoms.

5. Compositions according to one of claims 1 to 4, characterized in that as alcohol radicals it contains radicals (a) of n-dodecyl-1,12-diol, (b) of bis-(2-hydroxyethylether) or bis-(2-hydroxypropylether) of 2,2-isopropylidenediphenol or (c) of bis-(hydroxymethyl)-tricyclo-(5.2.1.$0^{2.6}$]-decane.

6. Use of compositions according to one of claims 1 to 5 for the preparation of dental compositions.

7. Use of a mixture of two compositions according to one of claims 2 to 5 in the molar ratio 1:1, which differ in that one composition x = 1 and in the other composition x = 2, for the preparation of dental compositions.

8. Use of compositions according to claim 6 or 7 in combination with polymerizable, ethylenic-unsaturated monomers, particularly with esters of (meth)acrylic acid and nono- or polyfunctional alcohols, particularly of the general formula $A(OH)_{n+1}$.

**Claims for the Contracting State: AT**

1. Use of (meth)acrylic acid esters for the preparation of dental compositions, characterized in that the (meth)acrylic acid esters have the general formula

$$(MO)_n\!-\!\!\left[A—O—CO—CH_2—O—CH_2—CH_2—O—CH_2—CO—O\right]_x\!A—(OM)_n \qquad \text{I}$$

wherein

A represents an alcohol radical with 4 to 30 chain-atoms between two points of linkage, where the chain-atoms are carbon or oxygen atoms,

M $CH_2{=}C(R^1)$—CO—, where $R^1$ represents hydrogen or methyl,

n represents 1 or 2,

x represents 0.3 to 3.

2. Use according to claim 1, characterized in that n in the general formula I has the value 1.

3. Use according to claim 1 or 2, characterized in that x in the general formula I has the value 0.5 to 2.

4. Use according to one of claims 1 to 3, characterized in that A in the general formula I has at least 8 chain-atoms.

5. Use according to one of claims 1 to 4, characterized in that the composition used contains as the alcohol radical, the radical (a) of n-dodecyl-1,12-diol, (b) of bis-(2-hydroxyethylether) or of bis-(2-hydroxypropylether) of 2,2-isopropylidenediphenol or (c) of bis-(hydroxymethyl)-tricyclo-(5.2.1.$0^{2.6}$]-decane.

6. Use according to one of claims 2 to 5, characterized in that a mixture of two compositions in the molar ratio 1:1 is used, which differ in that in one composition x = 1 and in the other x = 2.

7. Use according to one of claims 1 to 6, characterized in that it takes place in combination with polymerizable, ethylenic-unsaturated monomers, particularly with esters of (meth)acrylic acid and mono- or polyfunctional alcohols, particularly of the general formula $A(OH)_{n+1}$.

8. Process for the preparation of (meth)acrylic acid esters of the general formula

$$(MO)_n\!\left[A—O—CO—CH_2—O—CH_2—CH_2—O—CH_2—CO—O\right]_x\!A—(OM)_n \qquad \text{I}$$

wherein

A represents an alcohol radical with 4 to 30 chain-atoms between two points of linkage, where the chain-atoms are carbon or oxygen atoms,

M represents $CH_2=C(R^1)—CO—$, where $R^1$ represents hydrogen or methyl,
n represents 1 or 2,
x represents 0.3 to 3,

characterized in that a polyol of the general formula

$$A(OH)_{n+1}$$

wherein n represents the number 1 or 2, and A represents an alcohol radical with 4 to 30 chain-atoms between two points of linkage, and the chain-atoms are carbon or oxygen atoms, is partially esterified with triglycolic acid of the formula

$$HOOC—CH_2—O—CH_2—CH_2—O—CH_2—COOH$$

in the presence of an elasterification catalyst, and the remaining hydroxyl groups of the polyol are esterified with acrylic acid or methacrylic acid in the presence of a polymerisation inhibitor.

9. Process according to claim 8, characterized in that n has the value 1 and x has the value 0.5 to 2 in the general formula I.

10. Process according to claim 8 or 9, characterized in that A in the general formula I has at least 8 chain-atoms.